(19) Europäisches Patentamt
European Patent Office
Office européen des brevets

(11) Veröffentlichungsnummer: **0 031 954**
**B1**

(12) # EUROPÄISCHE PATENTSCHRIFT

(45) Veröffentlichungstag der Patentschrift: **14.03.84**

(51) Int. Cl.³: **C 07 C 143/72**, A 61 K 31/185

(21) Anmeldenummer: **80108207.4**

(22) Anmeldetag: **24.12.80**

(54) **Neue Sulfonamide, Verfahren zu deren Herstellung sowie diese Verbindungen enthaltende Arzneimittel.**

(30) Priorität: **07.01.80 DE 3000377**

(43) Veröffentlichungstag der Anmeldung: **15.07.81 Patentblatt 81/28**

(45) Bekanntmachung des Hinweises auf die Patenterteilung: **14.03.84 Patentblatt 84/11**

(84) Benannte Vertragsstaaten: **AT BE CH DE FR GB IT LI LU NL SE**

(56) Entgegenhaltungen:
**DE - A - 2 807 847**
**DE - A - 3 000 519**
**FR - A - 2 307 528**
**US - A - 4 112 236**

**JOURNAL OF MEDICINAL CHEMISTRY, Band 22, Nr. 7, 1979, Seiten 862-868 J.A. MONTGOMERY et al.: "Analogues of methotrexate"**
**JOURNAL OF MEDICINAL CHEMISTRY, Band 21, Nr. 7, Juli 1978, Seiten 673-677 G. NAIR et al.: "Folate analogues altered in the C9-N10 bridge region: N10-tosylisohomofolic acid and N10-tosylisohomoaminopterin"**
**CHEMICAL ABSTRACTS, Band 77, Nr. 17, 23. Oktober 1972, Seite 433, Nr. 114212r Columbus, Ohio, U.S.A. M. NATSUME et al.: "1,1-Disubstituted**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Witte, Ernst-Christian, Dr. rer nat., Beethovenstrasse 2, D-6800 Mannheim (DE)**
Erfinder: **Wolff, Hans Peter, Dr. rer nat., Rebenweg 24, D-6945 Hirschberg-Grosssachsen (DE)**
Erfinder: **Stegmeier, Karlheinz, Dr. rer. nat., Kirchbergstrasse 17, D-6148 Heppenheim (DE)**
Erfinder: **Roesch, Egon, Dr. med., Werderstrasse 44, D-6800 Mannheim (DE)**

(56) Entgegenhaltungen: (Fortsetzung)
**1,2,3,4-tetrahydroisoquinoline derivatives from isoquinolines"**

**EP 0 031 954 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Neue Sulfonamide, Verfahren zu deren Herstellung sowie diese Verbindungen enthaltende Arzneimittel

Die vorliegende Erfindung betrifft Sulfonamidoalkylphenylcarbonsäuren und ihre Derivate, Verfahren zu ihrer Herstellung sowie Arzneimittel, die diese Verbindungen enthalten.

Bekannt sind aus J. Med. Chem. 21, 673 ff. (1978) der 4-Tosylamidomethyl-benzoesäureethylester, aus J. Med. Chem. 22, 862 ff. (1979) weitere 4-Tosylamido- bzw. 4-Tosylamidomethyl-benzoesäure-Derivate und aus US 4 112 236 einige 4-Alkylsulfonamidoalkyl-benzoesäure-Derivate, insbesondere der 4-(3-Methansulfonamidopropyl)-benzoesäureethylester, als Zwischenprodukte ohne Hinweis auf eine pharmakologische Wirkung.

In der DE-OS 2 604 560 und in der DE-OS 2 532 420 sind Phenylcarbonsäuren, die in 4-Stellung durch eine Carbonamid-Gruppe substituiert sind, als blutzuckersenkende und lipidsenkende Substanzen beschrieben. Es wurde nun überraschenderweise gefunden, daß analoge Phenylcarbonsäuren, die in 4-Stellung durch eine Sulfonamid-Gruppe substituiert sind, sowohl eine ausgezeichnete lipidsenkende Wirkung als auch eine ausgeprägte Hemmwirkung auf die Thrombozytenaggregation zeigen.

Gegenstand der vorliegenden Erfindung sind daher neue Sulfonamide der allgemeinen Formel I

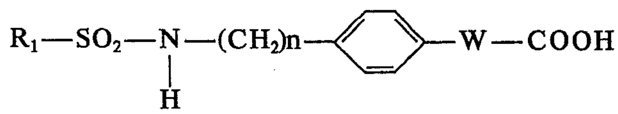

(I)

in welcher

- $R_1$ eine Aryl-, Aralkyl- oder Aralkenylgruppe, worin der Alkylrest der Aralkylgruppe 1–5 C-Atome und der Alkenylrest der Aralkenylgruppe 2–3 C-Atome enthält, und wobei der Arylrest jeweils einen aromatischen Kohlenwasserstoff mit 6–12 C-Atomen bedeutet, welcher gegebenenfalls ein- oder mehrfach durch Hydroxyl, Halogen, Trifluormethyl, Niederalkyl oder Niederalkoxy mit 1–5 C-Atomen oder Acetyl substituiert sein kann,
- n die Zahlen 2 und 3 bedeuten,
- W eine Bindung oder eine unverzweigte oder verzweigte Alkylenkette mit 1–4 C-Atomen darstellt, die entweder gesättigt ist oder eine Doppelbindung enthält,

sowie deren physiologisch unbedenklichen Salze, Ester und Amide.

Unter »niederem Alkyl« und »niederen Alkoxygruppen« sind sowohl geradkettige als auch verzweigte Reste zu verstehen. Vorzugsweise findet als geradkettiger niederer Alkylrest die Methylgruppe, als verzweigter niederer Alkylrest die tert.-Butylgruppe und als niedere Alkoxygruppe die Methoxygruppe Verwendung.

Als Aralkylreste kommen solche infrage, deren Alkylreste geradkettig oder verzweigt sind. Bevorzugt sind der Phenethyl- und der 4-Chlor-phenethylrest.

Als Aralkenylrest sind der Styryl- und der 4-Chlor-styrylrest bevorzugt.

Unter »Arylrest« sind insbesondere der Phenyl-, der Biphenylyl- und der Naphthylrest zu verstehen. Diese Arylreste können in allen möglichen Positionen wie angegeben substituiert sein.

Unter Halogen verstehe man vorzugsweise Fluor, Chlor und Brom.

Unter unverzweigten Alkylengruppen W seien die folgenden verstanden:

$-CH_2-$ $\quad -(CH_2)_2-$ und $-(CH_2)_3-$ (gesättigt)

sowie

$-CH{=}CH-$ (ungesättigt)

Als verzweigte Gruppen W werden bevorzugt beansprucht die Gruppen:

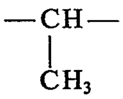 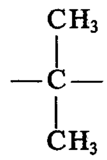 und 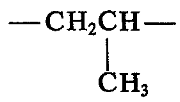 (gesättigt)

sowie

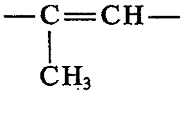 und 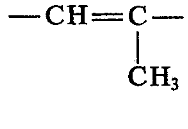 (ungesättigt).

Die von den Carbonsäuren der allgemeinen Formel I abgeleiteten Ester enthalten als Alkoholkomponente niedere einwertige Alkohole, von denen Methanol, Ethanol und n-Butanol bevorzugt sind, sowie mehrwertige Alkohole, z. B. Glykol oder Glycerin, oder Alkohole mit anderen funktionellen Gruppen, wie z. B. Ethanolamin oder Glykol-ether.

Die erfindungsgemäßen, von den Carbonsäuren der allgemeinen Formel I abgeleiteten Amide enthalten als Aminkomponente z. B. Ammoniak, p-Aminobenzoesäure, β-Alanin, Ethanolamin oder 2-Aminopropanol, wobei die bisher genannten bevorzugt sein sollen. Es kommen aber auch Alkylamine wie z. B. Isopropylamin oder tert.-Butylamin, Dialkylamine wie Diethylamin sowie cyclische Amine wie z. B. Morpholin oder 4-Alkyl- bzw. -Aralkyl- bzw. -Arylpiperazine, z. B. 4-Methylpiperazin, 4-(4-Chlorbenzyl)-piperazin oder 4-(3-Methoxyphenyl)-piperazin infrage.

Die obige Definition der erfindungsgemäßen Verbindungen soll auch alle möglichen Stereoisomeren sowie ihre Mischungen umfassen.

Gegenstand der vorliegenden Erfindung sind weiterhin Verfahren zur Herstellung von Sulfonamiden der allgemeinen Formel I, in der R, $R_1$, n und W die oben angegebene Bedeutung haben, indem man zu Beispiel

a) ein Amin der allgemeinen Formel II

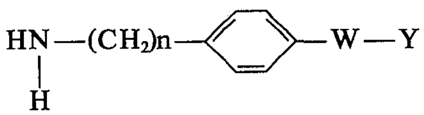

(II)

in welcher n und W hier und in allen folgenden Beispielen die oben angegebene Bedeutung haben und Y hier und in allen folgenden Beispielen die Gruppe $-COOR_2$, in der $R_2$ ein Wasserstoffatom oder eine niedere Alkylgruppe bedeutet, eine Säureamid-Gruppe oder einen Rest darstellt, der nach erfolgter Kondensation in die $COOR_2$-Gruppe oder in eine Säureamidgruppe überführt wird, in an sich bekannter Weise mit einer Sulfonsäure der allgemeinen Formel III

$$R_1-SO_2OH \qquad (III)$$

in welcher $R_1$ hier und in allen folgenden Beispielen die oben angegebene Bedeutung hat, bzw. einem Derivat derselben umsetzt, oder anstelle der freien Amine II deren Salze einsetzt; oder

– als eine Variante dieses Verfahrens – eine Umacylierung durchführt, die dann eintritt, wenn man eine freie Sulfonsäure III mit einer Verbindung der allgemeinen Formel IV

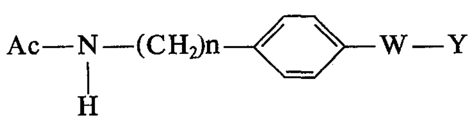

(IV)

in welcher Ac einen leicht austauschbaren Acylrest darstellt, in einem geeigneten Lösungsmittel umsetzt; oder

b) ein Sulfoamid der allgemeinen Formel V

$R_1-SO_2-NH$ (V)
H

mit einer Verbindung der allgemeinen Formel VI

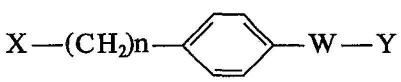

(VI)

umsetzt, wobei X hier und in allen folgenden Beispielen eine reaktive Gruppe darstellen soll, im Anschluß an die erfolgten Umsetzungen
gegebenenfalls die erhaltenen Säurederivate der allgemeinen Formel I in die freie Säure oder gewünschtenfalls die erhaltene freie Säure der allgemeinen Formel I in einen Ester, in ein Amid oder in ein physiologisch verträgliches Salz umwandelt; oder

c) eine Verbindung der allgemeinen Formel VII

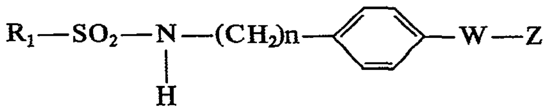 (VII)

in der Z einen oxidativ in die Carboxylgruppe überführbaren Rest darstellt, oxidiert.
Weitere Verfahren zur Herstellung der erfindungsgemäßen Verbindungen bestehen darin, daß man

d) eine Verbindung der allgemeinen Formel VIII

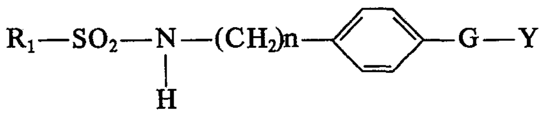 (VIII)

in welcher G eine Kohlenstoff-Kette darstellt, die eine der Gruppen

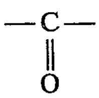 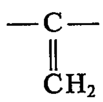 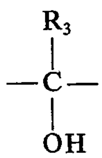 oder 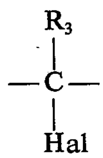

oder gegebenenfalls funktionelle Derivate dieser Gruppen enthält, reduziert; oder, für den Fall, daß W eine unverzweigte Kette darstellt,

e) eine Verbindung der allgemeinen Formel IX

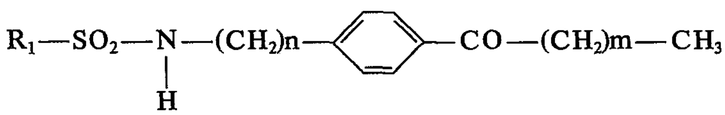 (IX)

in welcher m die Zahlen 0 bis 2 darstellt, unter den Bedingungen einer gegebenenfalls modifizierten Willgerodt-Kindler-Reaktion umsetzt.

f) Zur Herstellung von Verbindungen der allgemeinen Formel X

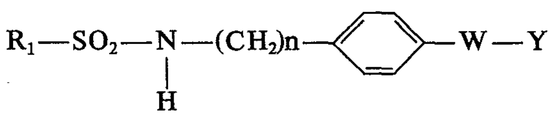 (X)

in der W die spezielle Bedeutung

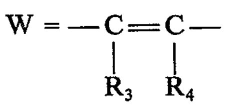

hat, kommen alle gängigen Verfahren zur Darstellung von Zimtsäuren und ihren Derivaten infrage:

f 1) Beim Vorliegen geeigneter Verbindungen X, in denen W die Bedeutung

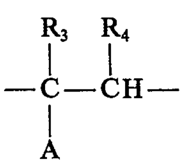 oder 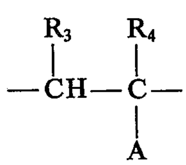

hat, wobei A ein Halogenatom, eine Hydroxygruppe oder eine funktionell abgewandelte Hydroxygruppe darstellt, gelangt man zu den gewünschten Verbindungen X, in denen W die Gruppe

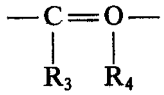

darstellt, durch Einwirkung eines HA abspaltenden Mittels.

f 2) Beim Vorliegen geeigneter Verbindungen XI

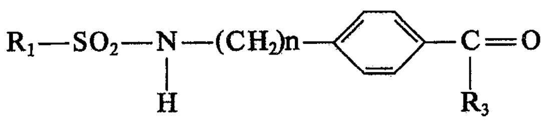

(XI)

kann man die gewünschten Zimtsäure-Derivate durch Reaktionen im Sinne einer Aldolkondensation, d. h. durch Umsetzen mit aktivierten CH-Gruppen, darstellen. Als solche Verbindungen kommen infrage Essigsäure bzw. deren Derivate, insbesondere aber Malonsäure-Derivate der allgemeinen Formel XII

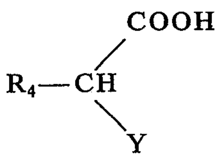

(XII)

wobei in letzterem Falle nach erfolgter Kondensation eine Decarboxylierung eintritt.
Als weitere Möglichkeit solcher aldolartiger Umsetzungen sei die Perkin-Reaktion genannt, die in einer Umsetzung von Verbindungen der allgemeinen Formel XI mit dem Anhydrid einer aliphatischen Carbonsäure in Gegenwart eines Alkalisalzes ggf. der gleichen Carbonsäure besteht.

f 3) Eine dritte Möglichkeit besteht in der Umsetzung von Verbindungen der allgemeinen Formel XI mit geeigneten phosphororganischen Verbindungen im Sinne modifizierter Wittig-Reaktionen. Als solche phosphororganischen Reaktionspartner seien z. B. die Alkoxycarbonylmethyl-phosphonsäurealkylester der allgemeinen Formel XIII genannt:

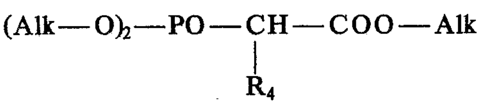

(XIII)

g) Durch Hydrieren der nach den Methoden f 1 bis f 3 oder nach anderen Methoden erhaltenen Zimtsäure-Derivate lassen sich die analogen Verbindungen mit gesättigter Kohlenstoff-Kette herstellen.

Als reaktive Derivate der Sulfonsäuren III kommen insbesondere die Halogenide sowie die Ester infrage. Die Umsetzungen der Sulfonsäurehalogenide mit Verbindungen der allgemeinen Formel II erfolgen zweckmäßig unter Zusatz eines säurebindenden Mittels, wie z. B. Alkaliacetat, Natriumhydrogencarbonat, Natriumcarbonat, Natriumphosphat, Calciumoxid, Calciumcarbonat oder Magnesiumcarbonat. Diese Funktion können aber auch organische Basen wie z. B. Pyridin oder Triethylamin übernehmen, wobei als inertes Lösungsmittel z. B. Ether, Benzol, Methylenchlorid, Dioxan oder ein Überschuß des tertiären Amins dient.

Bei Einsatz anorganischer Säurebinder verwendet man als Reaktionsmedium z. B. Wasser, wäßriges Ethanol oder wäßriges Dioxan.

Die Umacylierungsreaktion zwischen einer freien Sulfonsäure III und einem Acylamin IV wird unter Einsatz bevorzugt äquimolarer Mengen beider Reaktionspartner in einem polaren Lösungsmittel vorgenommen. Als solche polaren Lösungsmittel dienen z. B. Alkohole, insbesondere Ethanol und Methanol. Die Umsetzung verläuft bevorzugt bei der Siedetemperatur dieser Lösungsmittel. Als leicht austauschbarer Acylrest ist z. B. der Acetylrest zu nennen.

Zur Alkylierung der Sulfonamide V verwendet man bevorzugt Verbindungen VI, in denen X eine Arylsulfonyloxy-Gruppe darstellt. Bevorzugt soll X eine Toluolsulfonyloxy-Gruppe bedeuten. Als Alkylierungsmittel dienen also bevorzugt Arylsulfonsäurealkylester, eine Methode, die in ihrer Anwendung auf Sulfonsäureamide z. B. bei Klamann et al., Monatshefte für Chemie Bd. 83 (1952), S. 871, beschrieben ist. Die Umsetzungen erfolgen in alkalischem Milieu, bevorzugt ist als Reaktionsmedium heiße, konzentrierte Sodalösung.

Als oxidierbare Gruppen, welche in eine Carboxylfunktion überführt werden können, kommen vorzugsweise die Hydroxymethyl-, die Aminomethyl- und die Formylgruppe, ggf. auch die Acetylgruppe oder funktionelle Derivate dieser Gruppen infrage. Die Oxidation läßt sich mit den üblichen Oxidationsmitteln wie z. B. Mangan-IV-verbindungen, Permanganaten, Dichromaten, im Falle der Formylgruppe

auch mit Luftsauerstoff oder Silberoxid, im Falle der Acetylgruppe dagegen z. B. mit Hypobromit durchführen.

Zur Reduktion der in der allgemeinen Formel VIII enthaltenen Gruppen G eignen sich zahlreiche Verfahren. Die Reduktion der Gruppe —CO— kann z. B. nach Clemmensen mittels Zink/Salzsäure erfolgen. Bevorzugt ist jedoch die Reduktion mit Wasserstoff bei Normaldruck oder bei erhöhtem Druck in Gegenwart von Metallkatalysatoren wie z. B. Palladium oder Platin in Lösungsmitteln wie z. B. Essigsäure oder niederen Alkoholen.

Auch die Gruppen

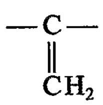 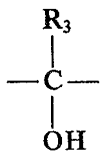 und 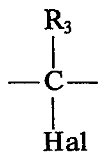

werden bevorzugt durch katalytisch angeregten Wasserstoff reduziert, wobei sich die eine Hydroxy-Gruppe enthaltende Gruppe G am besten in Gegenwart starker Säuren reduzieren läßt. Bevorzugt ist hierbei die Anwesenheit von Schwefelsäure oder Perchlorsäure in katalytischen Mengen. Gegebenenfalls kann auch mit komplexen Metallhydriden reduziert werden. Bevorzugt wird Natriumborhydrid eingesetzt. In diesem Fall kann die Umsetzung in einem Alkohol, insbesondere in Methanol, oder in Dioxan oder in wäßrig-alkalischem Milieu durchgeführt werden.

Die bei Verfahren e eingesetzten Ketone der allgemeinen Formel IX lassen sich leicht durch Friedel-Crafts-Acylierung darstellen. Sie werden mit Schwefel und einem sekundären Amin, bevorzugt mit Morpholin, umgesetzt. Das bei dieser »Willgerodt-Kindler-Reaktion« entstehende Thiomorpholid wird in an sich bekannter Weise mit Hilfe von starker Alkalilauge oder mit starker Salzsäure oder auch mit einem Gemisch aus Schwefelsäure, Eisessig und Wasser zur Carbonsäure verseift.

Zur Herstellung von Zimtsäure-Derivaten gemäß Verfahren f 1) eignen sich alle Verfahren, die eine Abspaltung von HA gestatten. Stellt A eine Hydroxygruppe dar, so kann eine Dehydratisierung mit den üblichen Agenzien wie z. B. Eisessig, Acetanhydrid, Schwefelsäure, Hydrogensulfat, Polyphosphorsäure, Phosphoroxichlorid, Thionylchlorid oder Phosphorpentoxid erfolgen, wobei zweckmäßigerweise in inerten Lösungsmitteln wie Benzol, Methylenchlorid, Tetrachlorkohlenstoff u. a. gearbeitet wird. Bevorzugt ist eine Dehydratisierung mit Phosphorpentoxid in siedendem Methylenchlorid. Die für die Dehydratisierung notwendigen Hydroxyverbindungen lassen sich z. B. durch eine Reformatzky-Reaktion aus den entsprechenden Aldehyden oder Ketonen darstellen, oder man gewinnt sie durch Reduktion der entsprechenden Ketoverbindungen entweder mit komplexen Hydriden wie z. B. Natriumborhydrid oder durch Hydrierung mit Raney-Nickel als Katalysator.

Zur Abspaltung von Halogenwasserstoff (wenn A ein Halogenatom bedeutet) kommen basische Agenzien infrage. Geeignete basische Agenzien sind z. B. anorganische oder organische Basen, wie NaOH, KOH, Natriumacetat, $Na_2CO_3$, $K_2CO_3$ sowie Alkoholate wie Natriummethylat, Amine wie z. B. Triethylamin, Dimethylanilin und Pyridin. Man arbeitet zweckmäßig in inerten Lösungsmitteln wie Dioxan, DMSO, Benzol, Petrolether oder Alkoholen wie Ethanol oder Isopropanol.

Die Kondensation von Verbindungen der allgemeinen Formel XI mit Malonsäure-Derivaten erfolgt in bekannter Weise durch Reaktion der beiden Partner in geeigneten Lösungsmitteln wie z. B. Pyridin, bevorzugt in Gegenwart eines primären oder sekundären Amins, wobei als sekundäres Amin Piperidin bevorzugt wird.

Die Umsetzungen zwischen Verbindungen XI mit Phosphonsäureestern (PO-aktivierte Olefinbildung nach Horner) wird in inerten Lösungsmitteln in Gegenwart von Basen vorgenommen. Als inerte Lösungsmittel eignen sich z. B. Diglyme, Benzol, Toluol, THF, DMF, aber auch Ether und Petrolether. Geeignete Basen sind z. B. Natriumamid, Organolithiumverbindungen, Alkoholate (meist im entsprechenden Alkohol gelöst), Natriumhydrid sowie Dimethylsulfoxylat in DMSO. Die Umsetzungen werden entweder bei Raumtemperatur oder bei erhöhten Temperaturen (Siedetemperatur des Lösungsmittels) durchgeführt.

Als Substituenten Y der allgemeinen Formel IV, die in die $-COOR_2$-Gruppe überführt werden können, kommen beispielsweise die Nitril-, Carbaldehyd-, Hydroxymethyl-, Aminomethyl- und Formylgruppe infrage.

Die gegebenenfalls in Anschluß an die Kondensation durchzuführende Umwandlung des Substituenten $R_2$ erfolgt beispielsweise durch Verseifung der Carbonsäureester ($R_2$ = Alkyl) zu den entsprechenden Carbonsäuren ($R_2$ = Wasserstoff) mit Mineralsäuren oder Alkalihydroxiden in einem polaren Lösungsmittel (wie Wasser, Methanol, Ethanol, Dioxan oder Aceton). Vorteilhaft wird die Verseifung mit einer starken Base (wie Natrium- oder Kaliumhydroxid) in einem Gemisch aus Methanol und Wasser bei Raumtemperatur oder bei mäßig erhöhten Temperaturen durchgeführt. Umgekehrt kann man aber auch die Carbonsäuren in üblicher Weise verestern oder Ester mit einem bestimmten Rest $R_2$ durch Umestern in einen Ester mit einem anderen Rest $R_2$ umwandeln. Die Veresterung der Carbonsäuren wird zweckmäßig in Gegenwart eines sauren Katalysators, wie z. B. Chlorwasserstoff, Schwefelsäure, p-Toluolsulfonsäure oder eines stark sauren Ionenaustauschharzes, vorgenommen. Umesterungen hin-

gegen erfordern den Zusatz einer geringen Menge einer basischen Substanz, z. B. eines Alkali- oder Erdalkalihydroxids oder eines Alkalialkoholats. Für die Veresterung der Carboxylgruppe bzw. für eine Umesterung eignen sich prinzipiell alle Alkohole. Bevorzugt sind die niederen einwertigen Alkohole wie Methanol, Ethanol oder Propanol, sowie mehrwertige Alkohole, z. B. Glykol, oder Alkohole mit anderen funktionellen Gruppen, wie Ethanolamin oder Glykolether.

Die erfindungsgemäßen, von den Carbonsäuren der allgemeinen Formel I abgeleiteten Amide werden bevorzugt nach an sich bekannten Methoden aus den Carbonsäuren oder ihren reaktiven Derivaten (wie z. B. Carbonsäurehalogeniden, -estern, -aziden, -anhydriden oder gemischten Anhydriden) durch Umsetzung mit Aminen hergestellt. Als Aminokomponenten kommen z. B. Ammoniak, Alkylamine, Dialkylamine, aber auch Aminoalkohole wie z. B. Ethanolamin und 2-Aminopropanol sowie Aminosäuren wie z. B. p-Aminobenzoesäure, $\beta$-Alanin und andere infrage. Andere wertvolle Aminkomponenten sind Alkyl-, Aralkyl- und Arylpiperazine.

Zur Herstellung von Salzen mit pharmakologisch verträglichen organischen oder anorganischen Basen, wie z. B. Natriumhydroxid, Kaliumhydroxid, Calciumhydroxid, Ammoniumhydroxid, Methylglukamin, Morpholin oder Ethanolamin können die Carbonsäuren mit den entsprechenden Basen umgesetzt werden. Auch Mischungen der Carbonsäuren mit einem geeigneten Alkalicarbonat bzw. -hydrogencarbonat kommen in Betracht.

Die neuen Verbindungen der allgemeinen Formel I und ihre physiologisch unbedenklichen Salze sowie ihre Ester und Amide zeigen sowohl eine ausgezeichnete lipidsenkende Wirkung als auch eine ausgeprägte Hemmwirkung auf die Thrombozytenaggregation.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z. B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z. B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z. B. Stärke, Lactose, Mannit, Methylcellulose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette oder feste hochmolekulare Polymere (wie Polyethylenglykole). Für die orale Applikation geeignete Zubereitungen können gewünschtenfalls Geschmacks- und Süßstoffe enthalten.

Die verabreichte Dosierung hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0,1 bis 50 mg/kg Körpergewicht. Normalerweise sind 0,5 bis 40 und vorzugsweise 1,0 bis 20 mg/kg/Tag in einer oder mehreren Anwendungen pro Tag wirksam, um die gewünschten Resultate zu erhalten.

Die überlegene Wirkung der erfindungsgemäßen Verbindungen gegenüber dem Handelspräparat Acetylsalicylsäure wird anhand der folgenden Versuche gezeigt:

## Versuchsprotokoll

### 1. Lipidsenkung

Jeweils 10 männlichen, stoffwechselgesunden Ratten wurde die zu testende Substanz 7 Tage in einer Dosis von 50 mg/kg in Methylcellulose-Suspension p.o. verabreicht. Am Versuchsende, 3 Stunden nach der letzten Sondierung wurden die Cholesterin- und Triglyceridwerte im Serum bestimmt. Die Veränderungen wurden im Vergleich zu Kontrollkollektiven ermittelt.

### 2. Aggregationshemmung

Die Beeinflussung der Thrombozyten-Aggregation wurde nach dem Born-Test vorgenommen:

#### a) Methodik

Venöses Blut von stoffwechselgesunden Probanden wird mit Natriumcitrat (9 : 1) gemischt. Durch Zentrifugation bei 150 g wurden die Erythrozyten sedimentiert. Im Überstand sind die Thrombozyten angereichert. Dieser Überstand ist als plättchenreiches Plasma (PRP) bezeichnet.

Ein Aliquot des PRP wird in die Küvette eines Aggregometers (Universal Aggregometer der Firma Braun Melsungen) gebracht und dort mittels eines kleinen Magneten gerührt. Die zu testende Substanz wird in wäßriger Lösung (pH ca. 7) zugesetzt. Veränderungen der Lichttransmission in der Suspension werden laufend durch einen Schreiber ausgezeichnet.

Nach Ablauf der spontanen Aggregation wird durch Zugabe von $5 \times 10^{-6}$ m Adrenalin in Aggregation ausgelöst. Es bilden sich größere Thrombozyten-Aggregate und dadurch nimmt die Lichttransmission durch die Suspension zu.

b) Auswertung

Die Adrenalin-induzierte Aggregation verläuft in 2 Phasen, d. h., die Lichttransmission nimmt zunächst zu, stagniert dann kurzfristig und nimmt nochmals zu. Durch Aggregationshemmer kann nur die 2. Phase der Aggregation beeinflußt werden.

Für die Dokumentation der Ergebnisse wird der Winkel der 2. Aggregationsphase gegen die Horizontale für die durch Adrenalin-induzierte Aggregation bestimmt und diese wird als 0%-Hemmung angesetzt (Kontrollversuch).

Mit dem gleichen PRP wird nach Zugabe der Testsubstanz die Aggregation mit Adrenalin induziert und der Verlauf der Aggregation über den Schreiber registriert. Der Winkel der 2. Phase gegenüber der Horizontalen wird wiederum bestimmt und das Verhältnis der beiden Winkel gibt die %-Hemmung der 2. Phase der Thrombozyten-Aggregation an.

Für das Vergleichspräparat Acetylsalicylsäure, beträgt die Hemmung bei einer Konzentration von $10^{-4}$ m 100%. Bei einer Konzentration von $5 \times 10^{-5}$ m 0%.

Alle Substanzen werden bei der Aggregation $5 \times 10^{-5}$ m geprüft.

| Substanz aus Beispiel | Lipidsenkung Triglyceride (%) | Cholesterin (%) | (%) Hemmung der adrenalin-induzierten Aggregation (bei $5 \times 10^{-5}$ m) |
|---|---|---|---|
| 1 | 24 | 7 | 0 |
| 1a | 9 | 13 | 100 |
| 1b | 0 | 10 | 0 |
| 1c | 38 | 7 | 100 |
| 1d | 10 | 10 | 0 |
| 3 | 34 | 8 | 100 |
| 3a | 0 | 20 | 0 |
| 3b | 10 | 3 | 0 |
| 3c | 30 | 7 | 50 |
| 3f | 24 | 5 | 100 |
| 3g | 20 | 10 | 30 |
| 4 | 10 | 10 | 100 |
| 4a | 31 | 11 | 100 |
| 4b, 7 | 39 | 16 | 20 |
| 4c | 0 | 12 | 0 |
| 4d, 6 | 42 | 19 | 100 |
| 4e | 20 | 6 | 100 |
| 4f | 14 | 0 | 100 |
| 4g | 10 | 0 | 100 |
| 4h | 22 | 0 | 50 |
| 4i | 27 | 0 | 100 |
| 4k | 50 | 25 | 50 |
| 4 l | 20 | 0 | 100 |
| 4m | 22 | 12 | 100 |

Bevorzugt im Sinne der Erfindung sind außer den in den Beispielen genannten Verbindungen und den durch Kombination aller in den Ansprüchen genannten Bedeutungen der Substituenten ableitbaren Verbindungen, noch insbesondere die folgenden Verbindungen:

4-(2-Benzolsulfonamidoethyl)phenylacetamid
3-{4-[2-(4-Phenylsulfonamido)ethyl]phenyl}propionsäure
3-{4-[2-(4-Phenylsulfonamido)ethyl]phenyl}propionamid

3-{4-[2-(4-Phenylsulfonamido)ethyl]phenyl}propionsäureethylester

Die nachfolgenden Beispiele zeigen einige der zahlreichen Verfahrensvarianten, die zur Synthese der erfindungsgemäßen Verbindungen verwendet werden können. Sie sollen jedoch keine Einschränkung des Erfindungsgegenstandes darstellen.

## Beispiel 1

### 4-[2-(2-Phenylethansulfonamido)ethyl]benzoesäure

Zu einer eisgekühlten Lösung von 14,3 g (70 mmol) 4-(2-Aminoethyl)benzoesäureethylester-hydrochlorid in 150 ml abs. Pyridin werden im Laufe einer Stunde unter Rühren 16,1 g (70 mmol) 2-Phenylethansulfochlorid zugetropft. Dann entfernt man das Kühlbad und rührt 2 Stunden bei Raumtemperatur. Anschließend gießt man in Eiswasser und säuert mit konzentrierter Salzsäure an, wobei sich ein Öl abscheidet, welches in Ether aufgenommen wird. Man extrahiert die wäßrige Phase noch mehrmals mit Ether, trocknet die vereinigten Etherphasen mit Natriumsulfat und dampft schließlich ein. Den Rückstand kristallisiert man aus einem Gemisch von Essigester und Ligroin um. Es bleiben 18,4 g (73% d. Th.) 4-[2-(2-Phenylethansulfonamido)ethyl]benzoesäureethylester mit dem Schmp. 83—86°C.

Man hält ein Gemisch aus 12,5 g (35 mmol) 4-[2-(2-Phenylethansulfonamido)ethyl]benzoesäureethylester, 70 ml 1 N Kalilauge und 200 ml Methanol 2 Stunden bei 35°C. Dann wird mit 2 N Salzsäure angesäuert. Man dampft das Methanol ab und extrahiert die zurückbleibende wäßrige Phase mehrmals mit Methylenchlorid. Die vereinigten Methylenchloridphasen werden mit Wasser gewaschen, über Natriumsulfat getrocknet und eingedampft. Man löst den Eindampfrückstand in Natriumhydrogencarbonatlösung und fällt durch Zugabe von 5 N Salzsäure aus. Die Kristalle werden abgesaugt und getrocknet. Es resultieren 9,3 g (88% d. Th.) 4-[2-(2-Phenylethansulfonamido)ethyl]benzoesäure mit dem Schmp. 183—186°C.

In Analogie dazu erhält man

a) 4-[2-(4-Chlorbenzolsulfonamido)ethyl]benzoesäureethylester

aus 4-(2-Aminoethyl)benzoesäureethylesterhydrochlorid
und 4-Chlorbenzolsulfochlorid
Schmp. 87—89°C; Ausbeute 84% d. Th.,

und daraus durch Hydrolyse

4-[2-(4-Chlorbenzolsulfonamido)ethyl]benzoesäure
Schmp. 199—201°C; Ausbeute 91% d. Th.

b) 4-{2-[2-(4-Chlorphenyl)ethansulfonamido]ethyl}phenylessigsäureethylester

aus 4-(2-Aminoethyl)phenylessigsäureethylesterhydrochlorid
und 2-(4-Chlorphenyl)ethansulfochlorid
Schmp. 69—70°C (wäßriges Ethanol); Ausbeute 76% d. Th.,

und daraus durch Hydrolyse

4-{2-[2-(4-Chlorphenyl)ethansulfonamido]ethyl}phenylessigsäure
Schmp. 155—156°C (wäßriges Ethanol); Ausbeute 67% d. Th.

c) 4-{2-[2-(4-Chlorphenyl)ethensulfonamido]ethyl}phenylessigsäureethylester

aus 4-(2-Aminoethyl)phenylessigsäureethylesterhydrochlorid
und 2-(4-Chlorphenyl)ethensulfochlorid
Schmp. 101—102°C (66proz. Ethanol); Ausbeute 70% d. Th.,

und daraus durch Hydrolyse

4-{2-[2-(4-Chlorphenyl)ethensulfonamido]ethyl}phenylessigsäure
Schmp. 175—176°C (wäßriges Ethanol); Ausbeute 87% d. Th.

d) 4-[2-(4-Chlorbenzolsulfonamido)ethyl]phenylessigsäureethylester

aus 4-(2-Aminoethyl)phenylessigsäureethylesterhydrochlorid und 4-Chlorbenzolsulfochlorid
Schmp. 88—90°C (Essigester + Ligroin); Ausbeute 96% d. Th.,

und daraus durch Hydrolyse

4-[2-(4-Chlorbenzolsulfonamido)ethyl]phenylessigsäure
Schmp. 138—140°C (Essigester + Ligroin); Ausbeute 93% d. Th.

Beispiel 2

4-(2-Methansulfonamidoethyl)benzoesäure

In ein Gemisch aus 150 ml abs. Pyridin, 12,8 g (92 mmol) pulverisiertem, wasserfreiem Kaliumcarbonat und 21,25 g (92 mmol) 4-(2-Aminoethyl)benzoesäureethylesterhydrochlorid tropft man bei 10—15°C 10,6 g (92 mmol) Methansulfochlorid. Anschließend wird 30 Minuten bei 20°C, dann 5 Minuten bei 80°C gerührt, danach abgekühlt und in Eiswasser gegossen. Man säuert mit konzentrierter Salzsäure an und extrahiert die ausgefallene Substanz mit Methylenchlorid. Nach Trocknen über Natriumsulfat wird die Methylenchloridphase eingedampft. Man erhält so 24,8 g (98% d. Th.) 4-(2-Methansulfonamidoethyl)benzoesäureethylester, Schmp. 96—99°C.
Durch Hydrolyse des Ethylesters in Analogie zu Beispiel 1 erhält man 93% d. Th. 4-(2-Methansulfonamidoethyl)benzoesäure mit dem Schmp. 173—174°C.

In Analogie dazu erhält man

a) 4-(2-Methansulfonamidoethyl)phenylessigsäureethylester

aus 4-(2-Aminoethyl)phenylessigsäureethylesterhydrochlorid und Methansulfochlorid
farbloses Öl; Ausbeute 81% d. Th.,

und daraus durch Hydrolyse

4-(2-Methansulfonamidoethyl)phenylessigsäure
Schmp. 170—172°C (Essigester + Ethanol); Ausbeute 79% d. Th.

b) 2-{4-[2-(2-Phenylethansulfonamido)ethyl]phenyl}propionsäureethylester

aus 2-[4-(2-Aminoethyl)phenyl]propionsäureethylester-hydrochlorid und 2-Phenylethansulfochlorid
farbloses Öl; Ausbeute 77% d. Th.

und daraus durch Hydrolyse

2-{4-[2-(2-Phenylethansulfonamido)ethyl]phenyl}propionsäure
Schmp. 94—97°C (Ether + Ligroin); Ausbeute 69% d. Th.

c) 2-{4-[2-(2-Phenylethansulfonamido)ethyl]phenyl}-2-methylpropionsäureethylester

aus 2-[4-(2-Aminoethyl)phenyl]-2-methylpropionsäureethylester-hydrochlorid und 2-Phenylethansulfochlorid
farbloses Öl; Ausbeute 52% d. Th.

und daraus durch Hydrolyse

2-{4-[2-(2-Phenylethansulfonamido)ethyl]phenyl}-2-methylpropionsäure
Schmp. 109—110°C (Ethanol + Wasser); Ausbeute 62% d. Th.

d) 3-⟨4-{2-[2-(4-Chlorphenyl)ethansulfonamido]ethyl}phenyl⟩propionsäureethylester

aus 3-[4-(2-Aminoethyl)phenyl]propionsäureethylester-hydrochlorid und 2-(4-Chlorphenyl)-ethansulfochlorid
Schmp. 70—71°C (Ethanol); Ausbeute 66% d. Th.

und daraus durch Hydrolyse

3-<4-{2-[2-(4-Chlorphenyl)ethansulfonamido]ethyl}phenyl>propionsäure
Schmp. 157–158°C (Ethanol + Wasser); Ausbeute 76% d. Th.

e) 3-{4-[2-(4-Chlorbenzolsulfonamido)ethyl]phenyl}propionsäureethylester

aus 3-[4-(2-Aminoethyl)phenyl]propionsäureethylester-hydrochlorid und 4-Chlorbenzolsulfochlorid
Schmp. 61–62°C (Ethanol); Ausbeute 65% d. Th.

und daraus durch Hydrolyse

3-{4-[2-(4-Chlorbenzolsulfonamido)ethyl]phenyl}propionsäure
Schmp. 129–130°C (Ethanol + Wasser); Ausbeute 87% d. Th.

f) 4-[2-(2-Phenylethansulfonamido)ethyl]zimtsäureethylester

aus 4-(2-Aminoethyl)zimtsäureethylester-hydrochlorid und 2-Phenylethansulfochlorid
Schmp. 98–99°C; Ausbeute 77% d. Th.

und daraus durch Hydrolyse

4-[2-(2-Phenylethansulfonamido)ethyl]zimtsäure
Schmp. 187–188°C; Ausbeute 76% d. Th.

g) 4-{2-[2-(4-Chlorphenyl)ethansulfonamido]ethyl}zimtsäureethylester

aus 4-(2-Aminoethyl)zimtsäureethylester-hydrochlorid und 2-(4-Chlorphenyl)ethansulfochlorid
Schmp. 91–92°C (Ethanol + Wasser); Ausbeute 63% d. Th.

und daraus durch Hydrolyse

4-{2-[2-(4-Chlorphenyl)ethansulfonamido)ethyl}zimtsäure
Schmp. 212–213°C (Ethanol + Wasser); Ausbeute 94% d. Th.

h) 4-[2-(4-Chlorbenzolsulfonamido)ethyl]zimtsäureethylester

aus 4-(2-Aminoethyl)zimtsäureethylester-hydrochlorid und 4-Chlorbenzolsulfochlorid
Schmp. 97–98°C (Ethanol); Ausbeute 92% d. Th.

und daraus durch Hydrolyse

4-[2-(4-Chlorbenzolsulfonamido)ethyl]zimtsäure
Schmp. 173–175°C; Ausbeute 93% d. Th.

i) 4-[2-(1-Naphthylsulfonamido)ethyl]zimtsäureethylester

aus 4-(2-Aminoethyl)zimtsäureethylester-hydrochlorid und 1-Naphthylsulfochlorid
Schmp. 85–86°C (Ethanol); Ausbeute 88% d. Th.

und daraus durch Hydrolyse

4-[2-(1-Naphthylsulfonamido)ethyl]zimtsäure
Schmp. 175°C (Ethanol + Wasser); Ausbeute 93% d. Th.

j) 4-[2-(2-Phenylethensulfonamido)ethyl]zimtsäureethylester

aus 4-(2-Aminoethyl)zimtsäureethylester-hydrochlorid und 2-Phenylethensulfochlorid
Schmp. 102–104°C; Ausbeute 63% d. Th.

und daraus durch Hydrolyse

4-[2-(2-Phenylethensulfonamido)ethyl]zimtsäure
Schmp. 190–191°C; Ausbeute 94% d. Th.

k) 4-{2-[2-(4-Chlorphenyl)ethensulfonamido]ethyl}zimtsäureethylester

aus 4-(2-Aminoethyl)zimtsäureethylester-hydrochlorid und 2-(4-Chlorphenyl)ethensulfochlorid Schmp. 131–132°C (Ethanol); Ausbeute 83% d. Th.

und daraus durch Hydrolyse

4-{2-[2-(4-Chlorphenyl)ethensulfonamido]ethyl}zimtsäure Schmp. 211–212°C (Ethanol + Wasser); Ausbeute 78% d. Th.

Beispiel 3

4-[2-(4-Toluolsulfonamido)ethyl]phenylessigsäure

Zu einer Lösung von 14,6 g (60 mmol) 4-(2-Aminoethyl)phenylessigsäureethylesterhydrochlorid in 120 ml abs. Pyridin tropft man bei 0–10°C in 5 Minuten ein Gemisch aus 12,0 g (63 mmol) 4-Toluolsulfochlorid und 50 ml Pyridin, läßt auf Raumtemperatur kommen und hält dann 45 Minuten auf 60°C. Man dampft anschließend im Vakuum bis zum halben Volumen ein, gießt in Eiswasser und säuert mit Salzsäure an. Die ausfallende viskose Masse wird in Essigester aufgenommen und die Lösung über Natriumsulfat getrocknet. Man dampft im Vakuum ein und kristallisiert den Rückstand aus einem Gemisch von Essigester und Ligroin um. Man erhält so 18,0 g (82%d. Th.) 4-[2-(4-Toluolsulfonamido)-ethyl]phenylessigsäureethylester mit dem Schmp. 113–115°C.

Zu einer Lösung von 13,3 g (37 mmol) 4-[2-(4-Toluolsulfonamido)ethyl]phenylessigsäureethylester in 220 ml Ethanol tropft man 110 ml 1 N Kalilauge und hält anschließend 2 Stunden bei 35–40°C. Dann wird im Vakuum das Ethanol abdestilliert und die wäßrige Phase mit Ether extrahiert. Zugabe von 55 ml 2 N Salzsäure führt zur Abscheidung eines farblosen Niederschlages, der abgesaugt und aus einem Gemisch von Essigester und Ligroin umkristallisiert wird. Ausbeute 10,8 g (88%d. Th.) 4-[2-(4-Toluolsulfonamido)ethyl]phenylessigsäure mit dem Schmp. 141–143°C.

In Analogie dazu erhält man

a) 4-[2-(2-Phenylethensulfonamido)ethyl]benzoesäureethylester

aus 4-(2-Aminoethyl)benzoesäureethylesterhydrochlorid und 2-Phenylethensulfochlorid Schmp. 59–61°C (Ethanol + Wasser); Ausbeute 66% d. Th.,

und daraus durch Hydrolyse

4-[2-(2-Phenylethensulfonamido)ethyl]benzoesäure Schmp. 169,5–170°C (Essigester); Ausbeute 81% d. Th.

b) 4-[2-(2-Phenylethansulfonamido)ethyl]phenylessigsäureethylester

aus 4-(2-Aminoethyl)phenylessigsäureethylesterhydrochlorid und 2-Phenylethansulfochlorid farbloses Öl; Ausbeute 61% d. Th.,

und daraus durch Hydrolyse

4-[2-(2-Phenylethansulfonamido)ethyl]phenylessigsäure Schmp. 150–152°C (Essigester); Ausbeute 69% d. Th.

c) 4-[2-(2-Phenylethensulfonamido)ethyl]phenylessigsäureethylester

aus 4-(2-Aminoethyl)phenylessigsäureethylesterhydrochlorid und 2-Phenylethensulfochlorid farbloses Öl; Ausbeute 78% d. Th.,

und daraus durch Hydrolyse

4-[2-(2-Phenylethensulfonamido)ethyl]phenylessigsäure Schmp. 146–149°C (Essigester + Ligroin); Ausbeute 81% d. Th.

d) 3-{4-[2-(2-Chlorbenzolsulfonamido)ethyl]phenyl}propionsäureethylester

aus 3-[4-(2-Aminoethyl)phenyl]propionsäureethylesterhydrochlorid und 2-Chlorbenzolsulfochlorid
Schmp. 57–60°C; Ausbeute 94% d. Th.,

und daraus durch Hydrolyse

3-{4-[2-(2-Chlorbenzolsulfonamido)ethyl]phenyl}propionsäure
Schmp. 136–139°C (Essigester + Ligroin); Ausbeute 82% d. Th.

e) 3-{4-[2-(3-Methoxybenzolsulfonamido)ethyl]phenyl}propionsäureethylester

aus 3-[4-(2-Aminoethyl)phenyl]propionsäureethylesterhydrochlorid und 3-Methoxybenzolsulfochlorid
farbloses Öl; Ausbeute 92% d. Th.,

und daraus durch Hydrolyse

3-{4-[2-(3-Methoxybenzolsulfonamido)ethyl]phenyl}propionsäure
Schmp. 100–103°C (mit Ether angerieben); Ausbeute 65% d. Th.

f) 3-{4-[2-(3-Trifluormethylbenzolsulfonamido)ethyl]phenyl}propionsäureethylester

aus 3-[4-(2-Aminoethyl)phenyl]propionsäureethylesterhydrochlorid und 3-Trifluormethylbenzolsulfochlorid
farbloses Öl; Ausbeute 99% d. Th.,

und daraus durch Hydrolyse

3-{4-[2-(3-Trifluormethylbenzolsulfonamido)ethyl]phenoxy}propionsäure
Schmp. 119–121°C (Toluol); Ausbeute 74% d. Th.

g) 3-<4-{2-[2-(4-Chlorphenyl)ethensulfonamido]ethyl}phenyl>propionsäureethylester

aus 3-[4-(2-Aminoethyl)phenyl]propionsäureethylesterhydrochlorid und 2-(4-Chlorphenyl)ethensulfonylchlorid
Schmp. 94–96°C (Essigester + Ligroin); Ausbeute 86% d. Th.,

und daraus durch Hydrolyse

3-<4-{2-[2-(4-Chlorphenyl)ethensulfonamido]ethyl}phenyl>propionsäure
Schmp. 165°C (Essigester + Methanol); Ausbeute 73% d. Th.

## Beispiel 4

### 4-[2-(4-Fluorbenzolsulfonamido)ethyl]phenylessigsäure

Man erwärmt ein Gemisch aus 11,0 g (51 mmol) 4-(2-Aminoethyl)phenylessigsäurehydrochlorid, 8,3 g (60 mmol) Kaliumcarbonat und 200 ml Wasser auf 80°C und gibt bei dieser Temperatur 9,5 g (49 mmol) 4-Fluorbenzolsulfochlorid zu. Danach wird weitere 2 Stunden bei 80°C gehalten, dann abgekühlt und mittels 2 N Salzsäure auf pH 2 gebracht. Man saugt den ausfallenden Niederschlag ab, trocknet ihn und kristallisiert aus 66proz. Ethanol um. Man erhält 10,2 g (62% d. Th.) 4-[2-(4-Fluorbenzolsulfonamido)ethyl]phenylessigsäure mit dem Schmp. 121–122°C.

In Analogie dazu erhält man

a) 3-[4-(2-Benzolsulfonamidoethyl)phenyl]propionsäure

aus 3-[4-(2-Aminoethyl)phenyl]propionsäurehydrochlorid und Benzolsulfochlorid
Schmp. 102,5–103°C (Essigester + Ligroin); Ausbeute 63% d. Th.

b) 4-(2-Benzolsulfonamidoethyl)benzoesäure

aus 4-(2-Aminoethyl)benzoesäurehydrochlorid und Benzolsulfochlorid
Schmp. 144,5–145°C (wäßriges Ethanol); Ausbeute 74% d. Th.

c) 4-[2-(4-Methoxybenzolsulfonamido)ethyl]benzoesäure

aus 4-(2-Aminoethyl)benzoesäurehydrochlorid und 4-Methoxybenzolsulfochlorid
Schmp. 177–178°C (wäßriges Ethanol); Ausbeute 68% d. Th.

d) 4-(2-Benzolsulfonamidoethyl)phenylessigsäure

aus 4-(2-Aminoethyl)phenylessigsäurehydrochlorid und Benzolsulfochlorid
Schmp. 127–128°C (Ligroin + Isopropanol); Ausbeute 92% d. Th.

e) 4-[2-(4-Methoxybenzolsulfonamido)ethyl]phenylessigsäure

aus 4-(2-Aminoethyl)phenylessigsäurehydrochlorid und 4-Methoxybenzolsulfochlorid
Schmp. 160–162°C (wäßriges Ethanol); Ausbeute 72% d. Th.

f) 4-[2-(4-Acetylbenzolsulfonamido)ethyl]phenylessigsäure

aus 4-(2-Aminoethyl)phenylessigsäurehydrochlorid und 4-Acetylbenzolsulfochlorid
Schmp. 193–194°C (wäßriges Ethanol); Ausbeute 82% d. Th.

g) 4-[2-(2-Naphthalinsulfonamido)ethyl]phenylessigsäure

aus 4-(2-Aminoethyl)phenylessigsäurehydrochlorid und 2-Naphthalinsulfochlorid
Schmp. 135–136°C (wäßriges Ethanol); Ausbeute 68% d. Th.

h) 4-(2-Benzolsulfonamidoethyl)zimtsäure

aus 4-(2-Aminoethyl)zimtsäurehydrochlorid und Benzolsulfochlorid
Schmp. 164–166°C (wäßriges Ethanol); Ausbeute 73% d. Th.

i) 4-(3-Benzolsulfonamidopropyl)benzoesäure

aus 4-(3-Aminopropyl)benzoesäurehydrochlorid und Benzolsulfochlorid
Schmp. 207,5–209°C (Aceton + Wasser); Ausbeute 63% d. Th.

j) 4-(3-Benzolsulfonamidopropyl)phenylessigsäure

aus 4-(3-Aminopropyl)phenylessigsäurehydrochlorid und Benzolsulfochlorid
Schmp. 128–129°C (Essigester + Ligroin); Ausbeute 84% d. Th.

k) 2-[4-(2-Benzolsulfonamidoethyl)phenyl]-2-methylpropionsäure

aus 2-[4-(2-Aminoethyl)phenyl]-2-methylpropionsäurehydrochlorid und Benzolsulfochlorid
Schmp. 86–88°C (Essigester); Ausbeute 80% d. Th.

l) 4-[2-(2,5-Dichlorbenzolsulfonamido)ethyl]phenylessigsäure

aus 3-[4-(2-Aminoethyl)phenylessigsäure-hydrochlorid und 2,5-Dichlorbenzolsulfochlorid
Schmp. 163–164°C (wäßriges Ethanol); Ausbeute 65% d. Th.

m) 4-[4-(2-Benzolsulfonamidoethyl)phenyl]buttersäure

aus 4-[4-(2-Aminoethyl)phenyl]buttersäurehydrochlorid und Benzolsulfochlorid
Schmp. 70–71°C (wäßriges Ethanol); Ausbeute 67% d. Th.

n) 2-[4-(2-Benzolsulfonamidoethyl)phenyl]propionsäure

aus 2-[4-(2-Aminoethyl)phenyl]propionsäure-hydrochlorid und Benzolsulfochlorid
Schmp. (Natriumsalz) 236–239°C; Ausbeute 83% d. Th.

o) 3-[4-(2-Benzolsulfonamidoethyl)phenyl]-2-methylpropionsäure

aus 3-[4-(2-Aminoethyl)phenyl]-2-methylpropionsäure-hydrochlorid und Benzolsulfochlorid
Schmp. 113–115°C (Essigester-Ligroin); Ausbeute 85% d. Th.

p) 4-(2-Benzolsulfonamidoethyl)α-methylzimtsäure

aus 4-(2-Aminoethyl)α-methylzimtsäure-hydrochlorid und Benzolsulfochlorid

Schmp. 147—148°C; Ausbeute 62% d. Th.

q) 4-[2-(2-Naphthylsulfonamido)ethyl]zimtsäure

aus 4-(2-Aminoethyl)zimtsäure-hydrochlorid und 2-Naphthylsulfochlorid
Schmp. 192—193°C (Ethanol + Wasser); Ausbeute 96% d. Th.

Beispiel 5

4-[2-(N-Methylbenzolsulfonamido)ethyl]phenylessigsäure

Zu einem Gemisch aus 10,4 g (30 mmol) 4-(2-Benzolsulfonamidoethyl)phenylessigsäureethylester (hergestellt durch Verestern der Säure gemäß Beispiel 4d mit Ethanol; Fp. 58—60°C), 60 ml Hexamethylphosphorsäuretriamid und 60 ml abs. Toluol gibt man 0,72 g (30 mmol) Natriumhydrid (als Mineralölsuspension) und rührt anschließend 2 Stunden bei 80°C. Dann wird abgekühlt, mit einem Gemisch aus 12,8 g (90 mmol) Methyljodid und 12 ml Hexamethylphosphorsäuretriamid versetzt, 15 Minuten bei 20°C gerührt und dann 3 Stunden bei 80°C gehalten. Nach dem Abkühlen gießt man auf Eis, bringt mittels Salzsäure auf pH 3 und extrahiert mehrmals mittels Toluol. Die Toluolphase wird eingedampft, der Eindampfrückstand mittels Kieselgel/Toluol chromatographiert. Man erhält 5,1 g (47% d. Th.) reinen 4-[2-(N-Methylbenzolsulfonamido)ethyl]phenylessigsäureethylester als farbloses Öl mit dem Brechungsindex $n_D^{20}$ = 1,5590.
Daraus erhält man durch Hydrolyse mittels 1 N Kalilauge in Ethanol:
4-[2-(N-Methylbenzolsulfonamido)ethyl]phenylessigsäure
Schmp. 159—160°C (Essigester); Ausbeute 63% d. Th.

Beispiel 6

Δ-(2-Benzolsulfonamidoethyl)phenylessigsäure

Zu einem eisgekühlten Gemisch aus 52,3 g (0,2 mol) N-(2-Phenylethyl)benzolsulfonamid, 80 ml 1,1,2,2-Tetrachlorethan und 40,8 g (0,52 mol) Acetylchlorid werden unter Rühren 88 g (0,664 mol) Aluminiumtrichlorid portionsweise zugegeben. Die Zugabe ist nach 1,5 Stunden beendet. Man läßt das Reaktionsgemisch noch 45 Minuten bei 0°C und erhitzt dann allmählich auf 80°C. Nach 1,5 Stunden gießt man auf Eis und trennt die organische Phase ab. Der wäßrige Anteil wird mit Methylenchlorid extrahiert, die vereinigten organischen Phasen über Natriumsulfat getrocknet und eingedampft. Der Rückstand wird mit 1 Liter 2 N Natronlauge unter Rühren 2 Stunden auf Rückflußtemperatur erhitzt. Nach dem Erkalten extrahiert man mit Methylenchlorid, trocknet die organische Phase über Natriumsulfat und dampft ein. Den Rückstand verreibt man mit Ether und saugt die Kristalle ab. Man erhält 31,3 g (52% d. Th.) 4-(2-Benzolsulfonamidoethyl)acetophenon, Schmp. 133—135°C.
Ein Gemisch von 9,4 g (31 mmol) 4-(2-Benzolsulfonamidoethyl)acetophenon, 1,6 g (50 mmol) Schwefel und 20 ml Morpholin werden unter Rühren 19 Stunden auf 135°C erhitzt. Nach dem Erkalten wird das Reaktionsgemisch in Wasser gegossen und die wäßrige Phase mit Methylenchlorid extrahiert. Die Methylenchloridphase wäscht man mit 1 N Salzsäure, trocknet sie über Natriumsulfat und dampft ein. Der Eindampfrückstand wird in 100 ml 1 N Natronlauge aufgenommen und die Lösung 2 Stunden auf Siedetemperatur erhitzt. Das erkaltete Reaktionsgemisch extrahiert man mit Essigester und fällt durch Zugabe von Salzsäure aus der wäßrigen Phase die 4-(2-Benzolsulfonamidoethyl)phenylessigsäure, die in allen physikalischen Eigenschaften mit der unter Beispiel 4d genannten Substanz identisch ist; Ausbeute 56% d. Th.

Beispiel 7

Δ-(2-Benzolsulfonamidoethyl)benzoesäure

Man löst 11,0 g (0,28 mol) Ätznatron in 90 ml Wasser, fügt 66 ml Dioxan zu und versetzt das Gemisch unter Rühren tropfenweise mit 16 g (0,1 mol) Brom. Dann kühlt man im Eisbad unter weiterem Rühren ab und gibt 10 g (0,033 mol) 4-(2-Benzolsulfonamidoethyl)ecetophenon zu. Nach 2 Stunden wird dann durch Zugabe von Salzsäure die Carbonsäure ausgefällt. Man kristallisiert aus wäßrigem Ethanol um und erhält 8,2 g (81%d. Th.) 4-(2-Benzolsulfonamidoethyl)benzoesäure, die in allen physikalischen Eigenschaften mit der unter Beispiel 4b genannten Substanz identisch ist.

## Beispiel 8

### 4-(2-Benzolsulfonamidoethyl)phenylessigsäure-[4-methylpiperazid]

Man erhitzt ein Gemisch aus 16,0 g (0,05 mol) 4-(2-Benzolsulfonamidoethyl)phenylessigsäure, 100 ml Benzol und 17,9 g (0,15 mol) Thionylchorid 5 Stunden auf Rückflußtemperatur. Dann werden Benzol und überschüssiges Thionylchlorid im Vakuum abdestilliert. Rohausbeute: quantitativ. Nach Umkristallisieren aus Toluol erhält man 14,9 g (88% d. Th.) 4-(2-Benzolsulfonamidoethyl)phenylacetylchlorid, Fp. 82°C (Zers.).

Zu einer eisgekühlten Lösung aus 3,0 g (30 mmol) N-Methylpiperazin und 100 ml abs. Pyridin werden im Laufe einer Stunde unter Rühren 10,1 g (30 mmol) 4-(2-Benzolsulfonamidoethyl)phenylacetylchlorid portionsweise zugefügt. Anschließend läßt man auf 20°C kommen, erwärmt 5 Stunden auf 90°C, kühlt ab und gießt in ca. 500 ml Eiswasser. Das Gemisch wird mit Methylenchlorid extrahiert und die Methylenchloridphase nach Trocknen über Natriumsulfat eingedampft. Den Rückstand nimmt man in Ether auf und fällt mit etherischer Salzsäure das Hydrochlorid. Man erhält nach Umkristallisieren aus Ethanol 8,7 g (66% d. Th.) 4-(2-Benzolsulfonamidoethyl)phenylessigsäure-[4-methylpiperazid]-hydrochlorid, Schmp. 168°C.

## Beispiel 9

### 4-[2-(4-Hydroxyphenylsulfonamido)ethyl]phenylessigsäure

Man versetzt eine Mischung aus 8,75 g (36 mmol) 4-(2-Aminoethyl)phenylessigsäureethylester und 90 ml Methylenchlorid mit 7,3 g (72 mmol) Triethylamin und rührt das Gemisch zunächst 1 Stunde bei Zimmertemperatur. Dann kühlt man im Eisbad ab und tropft langsam eine Lösung von 9,5 g (36 mmol) 4-Ethoxycarbonyloxybenzolsulfochlorid in 10 ml Methylenchlorid zu. Die Reaktionsmischung wird 1 Stunde bei 0°C, dann 3 Stunden bei Zimmertemperatur belassen. Anschließend wäscht man die organische Phase mit verdünnter Salzsäure und Wasser, trocknet und dampft ein.

Als Rückstand erhält man 15,0 g (96% d. Th.) 4-[2-(4-Ethoxycarbonyloxyphenylsulfonamido)ethyl]-phenyl-essigsäureethylester, farbloses Öl.

14,8 g (34 mmol) des rohen Esters werden mit 100 ml 2 N Natronlauge 2 Stunden auf Rückflußtemperatur erhitzt. Nach dem Erkalten klärt man die nunmehr entstandene Lösung mit Aktivkohle und fällt mit Salzsäure die 4-[2-(4-Hydroxyphenylsulfonamido)ethyl]phenyl-essigsäure aus. Schmp. 157—159°C (Essigester + Toluol); Ausbeute 67% d. Th.

## Beispiel 10

Es wurden Tabletten hergestellt: Jede Tablette enthält 10 mg 3-[4-(2-Benzolsulfonamidoethyl)phenyl]propionsäure. Die Tabletten wurden gemäß der folgenden Rezeptur hergestellt:

| | |
|---|---|
| 3-[4-(2-Benzolsulfonamidoethyl)phenyl]propionsäure | 10 g |
| Lactose | 80 g |
| Stärke | 29 g |
| Magnesiumstearat | 1 g |

Vorstehende Verbindung wurde fein pulverisiert und mit Lactose und Stärke vermischt. Das Gemisch wurde in herkömmlicher Weise granuliert. Magnesiumstearat wurde zu dem Granulat gegeben und das Gemisch zu 1000 Tabletten mit einem Einzelgewicht von 0,12 g verpreßt.

**Patentansprüche**

1. Sulfonamide der allgemeinen Formel I

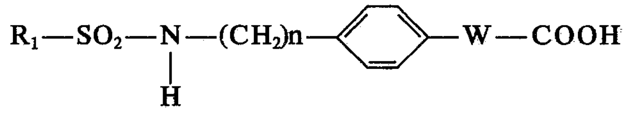

(I)

in welcher

$R_1$ eine Aryl-, Aralkyl- oder Aralkenylgruppe, worin der Alkylrest der Aralkylgruppe 1—5 C-Atome und der Alkenylrest der Aralkenylgruppe 2—3 C-Atome enthält, und wobei der Arylrest jeweils einen

aromatischen Kohlenwasserstoff mit 6—12 C-Atomen bedeutet, welcher gegebenenfalls ein- oder mehrfach durch Hydroxyl, Halogen, Trifluormethyl, Niederalkyl oder Niederalkoxy mit 1—5 C-Atomen oder Acetyl substituiert sein kann,

n die Zahlen 2 und 3 bedeuten,

W eine Bindung oder eine unverzweigte oder verzweigte Alkylenkette mit 1—4 C-Atomen darstellt, die entweder gesättigt ist oder eine Doppelbindung enthält,

sowie deren physiologisch unbedenklichen Salze, Ester und Amide.

2. Verbindungen nach Anspruch 1, dadurch gekennzeichnet, daß $R_1$ und W die angegebenen Bedeutungen haben und n die Zahl 2 bedeutet.

3. Verbindungen nach einem der Ansprüche 1 oder 2, dadurch gekennzeichnet, daß $R_1$ und n die angegebenen Bedeutungen haben und W eine Bindung bedeutet.

4. Verbindungen nach einem der Ansprüche 1 oder 2, dadurch gekennzeichnet, daß $R_1$ und n die angegebenen Bedeutungen haben und W eine der Gruppen $-CH_2-$, $-CH_2CH_2-$ oder $-C(CH_3)_2-$ bedeutet.

5. Verbindungen nach Anspruch 4, dadurch gekennzeichnet, daß n und W die angegebenen Bedeutungen haben und $R_1$ eine gegebenenfalls ein- oder mehrfach durch Chlor, Fluor, Trifluormethyl oder Methoxy substituierte Phenylgruppe bedeutet.

6. Verfahren zur Herstellung von Sulfonamiden der allgemeinen Formel I

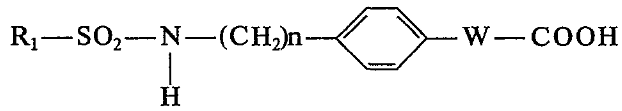

(I)

in welcher

$R_1$ eine Aryl-, Aralkyl- oder Aralkenylgruppe, worin der Alkylrest der Aralkylgruppe 1—5 C-Atome und der Alkenylrest der Aralkenylgruppe 2—3 C-Atome enthält, und wobei der Arylrest jeweils einen aromatischen Kohlenwasserstoff mit 6—12 C-Atomen bedeutet, welcher gegebenenfalls ein- oder mehrfach durch Hydroxyl, Halogen, Trifluormethyl, Niederalkyl oder Niederalkoxy mit 1—5 C-Atomen oder Acetyl substituiert sein kann,

n die Zahlen 2 und 3 bedeuten,

W eine Bindung oder eine unverzweigte oder verzweigte Alkylenkette mit 1—4 C-Atomen darstellt, die entweder gesättigt ist oder eine Doppelbindung enthält,

sowie deren physiologisch unbedenklichen Salze, Ester und Amide, dadurch gekennzeichnet, daß man

a) ein Amin der allgemeinen Formel II

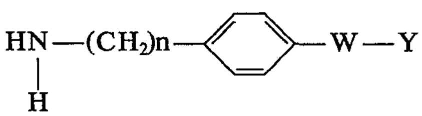

(II)

in welcher

n und W die oben angegebene Bedeutung haben und

Y die Gruppe $-COOR_2$, in der $R_2$ ein Wasserstoffatom oder eine niedere Alkylgruppe bedeutet, eine Säureamidgruppe oder einen Rest darstellt, der nach erfolgter Kondensation in die $COOR_2$-Gruppe oder in eine Säureamidgruppe überführt wird,

in an sich bekannter Weise mit einer Sulfonsäure der allgemeinen Formel III

$R_1—SO_2OH$ (III)

in welcher

$R_1$ die oben angegebene Bedeutung hat,

bzw. einem Derivat derselben umsetzt oder anstelle der freien Amine der allgemeinen Formel II deren Salze einsetzt;

oder

— als Variante dieses Verfahrens — eine Umacylierung durchführt, die dann eintritt, wenn man eine freie Sulfonsäure III mit einer Verbindung der allgemeinen Formel IV

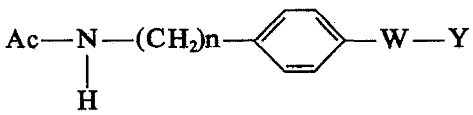

(IV)

in welcher

n, Y und W die oben angegebene Bedeutung haben und
Ac einen leicht austauschbaren Acylrest darstellt,

in einem geeigneten Lösungsmittel umsetzt;

oder

b) ein Sulfonamid der allgemeinen Formel V

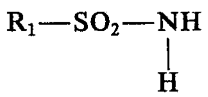

(V)

in welcher

$R_1$ die oben angegebene Bedeutung hat,

mit einer Verbindung der allgemeinen Formel VI

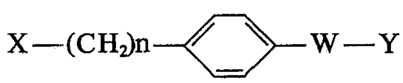

(VI)

in welcher

n und W die oben angegebene Bedeutung haben,
Y die Gruppe $-COOR_2$, in der $R_2$ ein Wasserstoffatom oder eine niedere Alkylgruppe bedeutet, eine Säureamidgruppe oder einen Rest darstellt, der nach erfolgter Kondensation in die $COOR_2$-Gruppe oder in eine Säureamidgruppe überführt wird, bedeutet und
X eine reaktive Gruppe darstellen soll,

umsetzt,

im Anschluß an die erfolgten Umsetzungen gegebenenfalls die erhaltenen Säurederivate der allgemeinen Formel I in die freie Säure oder gewünschtenfalls die erhaltene freie Säure der allgemeinen Formel I in einen Ester, in ein Amid oder in ein physiologisch verträgliches Salz umwandelt;

oder

c) eine Verbindung der allgemeinen Formel VII

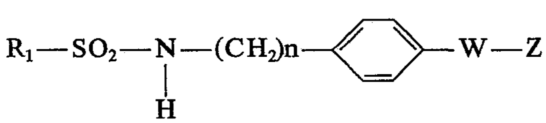

(VII)

in welcher

$R_1$, n und W die oben angegebene Bedeutung haben und
Z einen oxidativ in die Carboxylgruppe überführbaren Rest darstellt,

oxidiert.

7. Verbindungen der Formel I gemäß Anspruch 1 zur Erzielung einer thrombozytenaggregationshemmenden und/oder lipidsenkenden Wirkung.

8. Arzneimittel enthaltend eine Verbindung der Formel I gemäß Anspruch 1 sowie an sich bekannte

pharmakologisch unbedenkliche Träger- und Hilfsstoffe.

9. Verbindungen der Formel I gemäß Anspruch 1 zur Bekämpfung von Stoffwechselerkrankungen.

## Claims

1. Sulphonamides of the general formula (I)

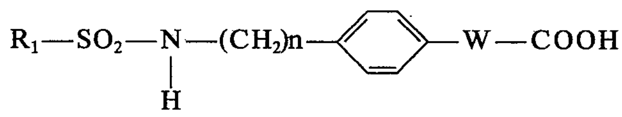

(I)

in which $R_1$ signifies an aryl, aralkyl or aralkenyl group, wherein the alkyl radical of the aralkyl group contains 1—5 C-atoms and the alkenyl radical of the aralkenyl group contains 2—3 C-atoms and whereby the aryl radical means, in each case, an aromatic hydrocarbon with 6—12 C-atoms, which can optionally be substituted one or more times by hydroxyl, halogen, trifluoromethyl, lower alkyl or lower alkoxy with 1—5 C-atoms or acetyl, n signifies the numbers 2 and 3, W represents a bond or an unbranched or branched alkylene chain with 1—4 C-atoms, which is either saturated or contains a double bond: as well as their physiologically acceptable salts, esters and amides.

2. Compounds according to claim 1, characterised in that $R_1$ and W have the given meanings and n signifies the number 2.

3. Compounds according to one of claims 1 or 2, characterised in that $R_1$ and n have the given meanings and W signifies a bond.

4. Compounds according to one of claims 1 and 2, characterised in that $R_1$ and n have the given meanings and W signifies one of the groups $-CH_2-$, $-CH_2CH_2-$ or $-C(CH_3)_2-$.

5. Compounds according to claim 4, characterised in that n and W have the given meanings and $R_1$ signifies a phenyl radical optionally substituted once or more times by chlorine, fluorine, trifluoromethyl or methoxy.

6. Process for the preparation of sulphonamides of the general formula I

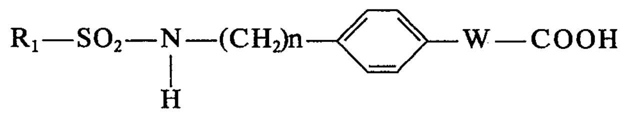

(I)

in which $R_1$ signifies an aryl, aralkyl or aralkenyl group, wherein the alkyl radical of the aralkyl group contains 1—5 C-atoms and the alkenyl radical of the aralkenyl group contains 2—3 C-atoms and whereby the aryl radical means, in each case, an aromatic hydrocarbon with 6—12 C-atoms, which can optionally be substituted one or more times by hydroxyl, halogen, trifluoromethyl, lower alkyl or lower alkoxy with 1—5 C-atoms or acetyl, n signifies the numbers 2 and 3, W represents a bond or an unbranched or branched alkylene chain with 1—4 C-atoms which is either saturated or contains a double bond, as well as of their physiologically acceptable salts, esters and amides thereof; characterised in that one

a) reacts an amine of the general formula II

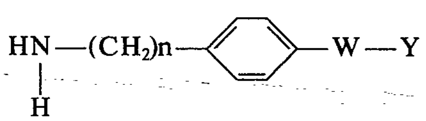

(II)

in which

n and W have the above-given meaning and

Y represents a $-COOR_2$ group, in which $R_2$ signifies a hydrogen atom or a lower alkyl group, an acid amide group or a residue which, after condensation has taken place, is converted into the $COOR_2$ group or into an acid amide group, in per se known manner with a sulphonic acid of the general formula III

$$R_1—SO_2OH \qquad (III)$$

in which

$R_1$ has the above-given meaning,

or with a derivative thereof or, instead of the free amine of the general formula II uses its salts; or—

as a variant of this process — carries out a transacylation which then takes place when one reacts a free sulphonic acid III with a compound of the general formula IV

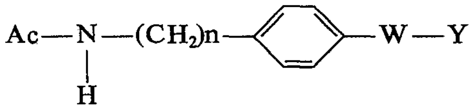 (IV)

in which

n, Y and W have the above-given meaning and
Ac represents an easily exchangeable acyl radical, in a suitable solvent;

or

b) reacts a sulphonamide of the general formula V

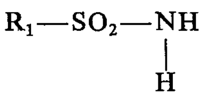 (V)

in which $R_1$ has the above-given meaning, with a compound of the general formula VI

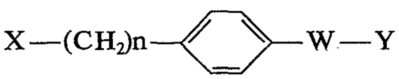 (VI)

in which

n and W have the above-given meaning,
Y represents a $-COOR_2$ group, in which $R_2$ signifies a hydrogen atom or a lower alkyl group, an acid amide group or a residue which, after condensation has taken place, is converted into the $COOR_2$ group or into an acid amide group and
X is to represent a reactive group; subsequent to the reactions having taken place optionally converts the acid derivative obtained of general formula I into an ester, into an amide or into a physiologically acceptable salt;

or

c) oxidises a compound of general formula VII

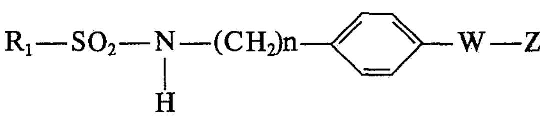 (VII)

in which

$R_1$, n and W have the above-given meaning and
Z represents a residue oxidatively convertible into the carboxyl group.

7. Compounds of formula I according to claim 1 for the achievement of a thrombocyte aggregation-inhibiting and/or lipid-sinking action.
8. Pharmaceutical compositions containing a compound of formula I according to claim 1, as well as per se known pharmacologically acceptable carrier and adjuvant materials.
9. Compounds of formula I according to claim 1 for the combating of metabolic diseases.

**Revendications**

1. Sulfonamides de formule générale I

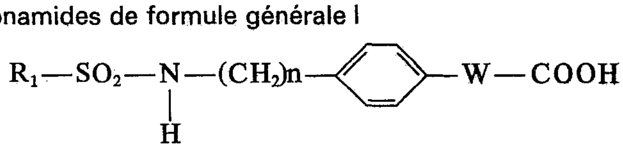 (I)

dans laquelle

$R_1$ est un groupe aryle, aralkyle ou aralcényle, dans lesquels le reste alkyle du groupe aralkyle possède de 1 à 5 atomes de carbone et le reste alcényle du groupe aralcényle 2 ou 3 atomes de carbone, le reste aryle étant chaque fois un hydrocarbure aromatique ayant de 6 à 12 atomes de carbone, éventuellement substitué une ou plusieurs fois par de l'hydroxyle, de l'halogène, du trifluorométhyle, de l'alkyle inférieur ou de l'alcoxy inférieur ayant de 1 à 5 atomes de carbone ou par de l'acétyle,
n est le nombre 2 ou 3,
W représente une liaison ou une chaîne alkylène droite ou ramifiée ayant de 1 à 4 atomes de carbone, cette chaîne étant saturée ou comportant une double liaison,

ainsi que leurs sels, esters et amides physiologiquement irréprochables.

2. Composés selon la revendication 1, caractérisés en ce que $R_1$ et W ont les significations indiquées et n est le nombre 2.

3. Composés selon la revendication 1 ou 2, caractérisés en ce que $R_1$ et n ont les significations indiquées et W représente une liaison.

4. Composés selon la revendication 1 ou 2, caractérisés en ce $R_1$ et n ont les significations indiquées et W est un des groupes $-CH_2-$, $-CH_2CH_2-$ et $-C(CH_3)_2$.

5. Composés selon la revendication 4, caractérisés en ce que n et W ont les significations indiquées et $R_1$ est un groupe phényle éventuellement substitué une ou plusieurs fois par du chlore, du fluor, du trifluorométhyle ou du méthoxy.

6. Procédé de préparation de sulfonamides de formule générale I

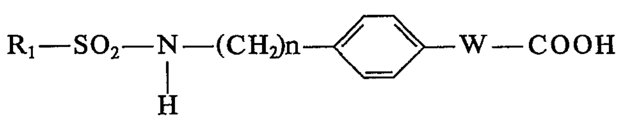

(I)

dans laquelle

$R_1$ est un groupe aryle, aralkyle ou aralcényle dans les quels le reste alkyle du groupe aralkyle possède de 1 à 5 atomes de carbone et le reste alcényle du groupe aralcényle possède 2 ou 3 atomes de carbone, le reste aryle étant chaque fois un hydrocarbure aromatique ayant de 6 à 12 atomes de carbone, pouvant être substitué éventuellement une ou plusieurs fois par de l'hydroxyle, de l'halogène, du trifluorométhyle, de l'alkyle inférieur ou de l'alcoxy inférieur ayant de 1 à 5 atomes de carbone ou par de l'acétyle,
n est le nombre 2 ou 3
W est une liaison ou une chaîne alkylène droite ou ramifiée ayant de 1 à 4 atomes de carbone, cette chaîne étant saturée ou comportant une double liaison,

ainsi que leurs sels, esters et amides physiologiquement irréprochables, caractérisé en ce que:

a) on fait réagir une amine de formule générale II

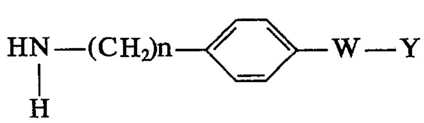

(II)

dans laquelle

n et W ont la signification ci-dessus indiquée et
Y est le groupe $-COOR_2$, dans lequel $R_2$ est un atome d'hydrogène ou un groupe alkyle inférieur, ou est un groupe amide ou un reste qui est transformé après l'achèvement de la condensation en ce groupe $-COOR_2$ ou en un groupe amide,

de façon en soi connue avec un acide sulfonique de formule générale III

$R_1-SO_2OH$ (III)

dans laquelle

$R_1$ a la signification ci-dessus indiquée,

ou avec un dérivé de cet acide, ou bien on remplace l'amine libre de formule générale II par un de ses sels;

ou

— selon une variante de ce procédé — ou réalise une transacylation, qui se produit lorsqu'on fait réagir un acide sulfonique III libre avec un composé de formule générale IV

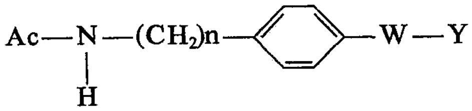

(IV)

dans laquelle

n, Y et W ont la signification ci-dessus indiquée et
Ac est un reste acyle facilement échangeable,

dans un solvant approprié;

ou

b) on fait réagir un sulfonamide de formule générale V

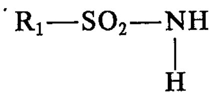

(V)

dans laquelle

$R_1$ a la signification ci-dessus indiquée,
avec un composé de formule générale VI

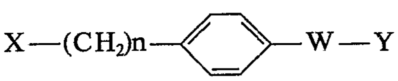

(VI)

dans laquelle

n et W ont la signification donnée ci-dessus,
Y est le groupe $-COOR_2$, dans lequel $R_2$ est un atome d'hydrogène ou un groupe alkyle inférieur, ou est un groupe amide ou un reste qui est transformé après l'achèvement de la condensation en ce groupe $-COOR_2$ ou en un groupe amide, et,
X est un groupe réactif,

et consécutivement à l'achèvement de ces réactions, on transforme éventuellement les dérivés d'acide de formule générale I obtenus en acides libres ou, si on le désire, on transforme les acides libres de formule générale I obtenus en un ester, un amide ou un sel physiologiquement tolérable;

ou

c) on oxyde un composé de formule générale VII

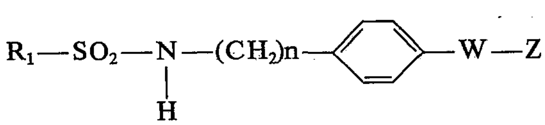

(VII)

dans laquelle

$R_1$, n et W ont la signification donnée ci-dessus et
Z est un reste pouvant être transformé en groupe carboxyle par oxydation.

7. Composés de formule I selon la revendication 1 pour l'obtention d'une activité d'inhibition de l'agglutination des thrombocytes et/ou d'une activité réductrice du taux de lipides.
8. Médicament contenant un composé de formule I selon la revendication 1, ainsi que des substances de support et auxiliaires connues, pharmacologiquement irréprochables.
9. Composés de formule I selon la revendication 1 pour le traitement des maladies du métabolisme.